# EUROPÄISCHE PATENTSCHRIFT

(11) **EP 1 503 855 B1**
(45) Veröffentlichungstag und Bekanntmachung des Hinweises auf die Patenterteilung: **13.04.2011**
(21) Anmeldenummer: 03729967.4
(22) Anmeldetag: 02.05.2003
(51) Int. Cl.: B01J 21/06, B01J 37/00, B01J 23/24, B01J 23/40, B01J 23/70, C07C 5/333, C07C 5/327

(54) **Katalysator mit Träger auf Basis von Zirkondioxid und Verwendung für Dehydrierungen**
Catalyst with zirconia based carrier and its use for dehydrogenation reactions
Catalyseur avec support à base de zircone et son utilisation dans des réactions de déshydrogénation

(30) Priorität: 03.05.2002 DE 10219879
(43) Veröffentlichungstag der Anmeldung: 09.02.2005
(73) Patentinhaber: BASF SE, 67056 Ludwigshafen (DE)
(72) Erfinder: HOFSTADT, Otto, 67346 Speyer (DE); HESSE, Michael, 67549 Worms (DE); SCHINDLER, Götz-Peter, 68219 Mannheim (DE); HARTH, Klaus, 67317 Altleiningen (DE); SIMON, Falk, 64625 Bensheim (DE)
(86) Internationale Anmeldenummer: PCT/EP2003/004625
(87) Internationale Veröffentlichungsnummer: WO 2003/092887

(56) Entgegenhaltungen:
- EP-A- 0 700 718
- EP-A- 1 074 301
- WO-A-02/051547
- FR-A- 2 590 887
- GB-A- 1 356 249
- US-A- 4 631 267

## Beschreibung

Die Erfindung betrifft einen Katalysator, der einen Träger umfaßt, sowie die Verwendung als Dehydrierkatalysator.

Es ist bekannt, Zircondioxid als Katalysatorträger für Dehydrierkatalysatoren einzusetzen.

EP-A 0 716 883 offenbart einen Katalysatorträger, der im wesentlichen aus monoklinem Zircondioxid besteht. Dieser wird durch Zugabe einer Zirconylnitrat- oder Zirconylchlorid-Lösung zu einer wässrigen Ammoniaklösung, wobei der pH-Wert von 14 auf 6 absinkt, Auswaschen des Fällproduktes, Trocknen, Calcinieren und Tablettieren hergestellt. Der so hergestellte Katalysatorträger besteht zu 85 bis 100 Gew.-% aus monoklinem Zircondioxid.

DE-A 196 54 391 beschreibt die Herstellung eines Dehydrierkatalysators durch Tränkung von im wesentlichen monoklinem ZrO₂ mit einer Lösung von Pt(NO₃)₂ und Sn(OAc)₂ bzw. durch Tränkung des ZrO₂ mit einer ersten Lösung von Cr(NO₃)₃ und anschließend einer zweiten Lösung von La(NO₃)₃. Die imprägnierten Träger werden getrocknet und anschließend calciniert. Die so erhaltenen Katalysatoren werden als Dehydrierkatalysatoren für die Dehydrierung von Propan zu Propen eingesetzt.

EP-A 0 700 718 beschreibt ein Verfahren zur Herstellung eines Katalysators oder Katalysatorträgers, wobei eine Mischung gebildet wird, die u.a. Rohmasse, ausgewählt aus keramischem Material und/oder Molekularsieb, und Silikonharz als Bindemittel enthält. Diese wird zu einem Grünkörper verformt, getrocknet und thermisch behandelt.

EP-A 1 074 301 beschreibt Katalysatoren mit bimodaler Porenradienverteilung und einer BET-Oberfläche von größer als 70 m²/g die a) 10 bis 99,9 Gew.-% Zirkondioxid und b) 0 bis 60 Gew.-% Aluminiumoxid, Siliciumdioxid und/oder Titandioxid und c) 0,1 bis 10 Gew.-% mindestens eines Elementes der ersten oder zweiten Hauptgruppe, eines Elementes der dritten Nebengruppe, eines Elementes der achten Nebengruppe des Periodensystems der Elemente, Lanthan und/oder Zinn enthalten. EP-A 1 074 301 beschreibt auch ein Verfahren zur Herstellung der Katalysatoren, wobei man der Katalysatorrohmasse 2 bis 30 Gew.-% Polyamine, Polyacrylate, Polyalkohole, Polysiloxane, Kohlenhydrate oder deren Gemische zusetzt und diese oberhalb von 550 °C calciniert. Die Katalysatoren werden für die Dehydrierung von Kohlenwasserstoffen verwendet.

Die Katalysatoren des Standes der Technik sind hinsichtlich ihrer Aktivität und ihrer Standzeit noch verbesserungsfähig.

Aufgabe der vorliegenden Erfindung ist es, Dehydrierkatalysatoren mit verbesserten Eigenschaften, insbesondere mit verbesserter Aktivität des Katalysators, bereitzustellen.

Gelöst wird die Aufgabe durch einen katalysator gemäß Anspruch 1. Erfindungsgemäß wurde gefunden, daß sich durch Vermischen von üblicherweise im wesentlichen monoklinem Zircondioxidpulver, welches eine hohe Oberfläche aufweist, mit einer Organosiliciumverbindung als Bindemittel, welche beim Calcinieren SiO₂ bildet, Formung zu Formkörpern wie Tabletten, Strängen und Kugeln und Calcinieren der Formkörper, Katalysatorträger mit hoher mechanischer Stabilität und einer für die Dehydrierung von Alkanen sehr gut geeigneten Porenstruktur herstellen lassen. Die Träger der erfindungsgemäßen Katalysatoren sind ausreichend stabil, um mehrere hundert oxidative Regenerierzyklen ohne mechanische Schädigung und Aktivitätsverlust zu überstehen.

Die als Bindemittel eingesetzten Organosiliciumverbindungen sind im allgemeinen flüssig. Dadurch wird das hochoberflächige Zircondioxid beim Vermischen gleichmäßig mit der Organosiliciumverbindung benetzt, wodurch die Zircondioxidpartikel von der Organosiliciumverbindung umschlossen und teilweise durchtränkt werden. Daraus resultiert eine hohe Bindungsverstärkung zwischen den Zircondioxidpartikeln und eine sehr gute mechanische Stabilität der erhaltenen Katalysatorträger-Formkörper. Beim Calcinieren der Katalysatorträger-Formkörper verbrennen die organischen Reste des siliciumorganischen Bindemittels. Dabei wird SiO₂ gebildet, welches in der Zircondioxidmatrix sehr fein verteilt vorliegt. Durch die Verbrennung der organischen Reste des siliciumorganischen Bindemittels entstehen zusätzliche Poren. Diese Poren sind aufgrund der gleichmäßigen Verteilung des siliciumorganischen Bindemittels in der Zircondioxidmatrix ebenfalls sehr gleichmäßig verteilt. Dadurch wird die Gesamtporosität des Katalysatorträgers erhöht. Durch die Gegenwart von SiO₂ wird zudem eine Stabilisierung des Zircondioxids gegen thermische Sinterung bewirkt. Diese ist um so ausgeprägter, je gleichmäßiger das Siliciumdioxid verteilt ist.

Das siliciumorganische Bindemittel wird ausgewählt aus monomeren Verbindungen der nachstehenden allgemeinen Formeln (I) bis (VI):

(Hal)ₓSiR₄₋ₓ (I)

(Hal)ₓSi(OR¹)₄₋ₓ (II)

(Hal)ₓSi(NR¹R²)₄₋ₓ (III)

RₓSi(OR¹)₄₋ₓ (IV)

RₓSi(NR¹R²)_{4-X} (V)

(R¹O)ₓSi(NR¹R²)₄₋ₓ (VI)

worin
- Hal: unabhängig voneinander Halogen (F, Cl, Br oder I),
- R: unabhängig voneinander H oder einen gegebenenfalls substituierten Alkyl-, Alkenyl-, Alkinyl-, Cycloalkyl-, Cycloalkenyl-, Arylalkyl- oder Arylrest,
- R¹, R²: unabhängig voneinander H oder einen gegebenenfalls substituierten Alkyl-, Acyl-, Arylalkyl- oder Arylrest, und
- X: 0 bis 4
bedeuten.

R, R¹ und R² können H, einen Alkylrest, vorzugsweise einen C₁- bis C₆-Alkylrest, der linear oder verzweigt sein kann, bedeuten. Falls R ein Alkylrest ist, ist R insbesondere Methyl, Ethyl, n-Propyl, iso-Propyl, n-Butyl, sec.-Butyl oder tert.-Butyl, speziell Methyl oder Ethyl. R, R¹ und R² können weiterhin einen Arylrest, vorzugsweise Phenyl oder einen Arylalkylrest, vorzugsweise Benzyl, bedeuten.

R kann darüber hinaus einen Alkenylrest, vorzugsweise einen C₂-C₆-Alkenylrest, insbesondere Vinyl oder Allyl, oder einen Alkinylrest, vorzugsweise Ethinyl, bedeuten.

R¹ und R² können darüber hinaus einen Acylrest, vorzugsweise einen C₂-C₆-Acylrest, insbesondere einen Acetylrest, bedeuten.

Beispiele für geeignete Organosiliciumverbindungen der allgemeinen Formel (I) sind beispielsweise SiCl₄, MeSiCl₃, Me₂SiCl₂ und Me₃SiCl.

Geeignete Organosiliciumverbindungen der allgemeinen Formel (IV) sind beispielsweise Si(OEt)₄, MeSi(OEt)₃, Me₂Si(OEt)₂ und Me₃SiOEt.

Geeignete Verbindungen der allgemeinen Formel (V) sind beispielsweise Me₃Si(NMeCOMe) und MeSi(NMeCOCH₂C₆H₅).

Eine geeignete Verbindungen der allgemeinen Formel (VI) ist beispielsweise (MeO)₃SiNMe₂.

In einem ersten Schritt des Verfahrens vermischt man Zircondioxidpulver mit dem siliciumorganischen Bindemittel, gegebenenfalls einem Porenbildner, gegebenenfalls einer Säure, Wasser sowie gegebenenfalls weiteren Zuschlagstoffen zu einer knetbaren Masse. Vorzugsweise werden
- a): 50 bis 98 Gew.-% Zircondioxidpulver,
- b): 2 bis 50 Gew.%, besonders bevorzugt 5 bis 20 Gew.% der Organosiliciumverbindung,
- c): 0 bis 48 Gew.-%, besonders bevorzugt 0 bis 10 Gew.-% Porenbildner, und
- d): 0 bis 48 Gew.-%, besonders bevorzugt 0 bis 10 Gew.-% weitere Zuschlagstoffe, wobei die Summe der Komponenten a) bis d) 100 Gew.-% ergibt, unter Zusatz von Wasser und einer Säure zu einer knetbaren Masse vermischt.

Das Zircondioxidpulver ist Zircondioxidpulver mit einer hohen Oberfläche, üblicherweise im wesentlichen monoklines Zircondioxidpulver. Im wesentlichen monoklines Zircondioxidpulver, das zu 85 bis 100 Gew.-%, bevorzugt zu 90 bis 100 Gew.% aus monoklinem Zircondioxid besteht, kann wie in EP-A 0 716 883 beschrieben, durch Fällung von Zirconiumsalzen mit Ammoniak hergestellt werden, indem man eine Zirconylnitrat- oder Zirconylchlorid-Lösung zu einer wässrigen Ammoniaklösung zugibt, wobei der pH-Wert von 14 auf 6 absinkt, das Fällungsprodukt auswäscht, trocknet und calciniert. Dazu stellt man zunächst aus Zirconcarbonat und Salzsäure eine möglichst konzentrierte, in der Regel 2 bis 5 mol-%ige Zirconchlorid-Lösung oder bevorzugt aus Zirconcarbonat und Salpetersäure eine möglichst konzentrierte, in der Regel 2 bis 5 mol-%ige Zirconnitrat-Lösung her. Diese Lösung wird in der Regel bei Temperaturen von 20 bis 60 °C unter Kontrolle des pH-Wertes zu einer vorgelegten wässrigen Ammoniak-Lösung (ca. 15 mol-% NH₃) zugegeben, wobei die Zugabe bei einem pH-Wert von 6 bis 8 beendet wird und der pH-Wert nicht unter 6 fallen darf. Daran schließt sich eine Nachrührzeit von im allgemeinen 30 bis 600 min an.

Das Fällungsprodukt wird beispielsweise auf einer Filterpresse ausgewaschen und von Ammoniumsalzen im wesentlichen befreit, getrocknet und bei einer Temperatur von 300 bis 600 °C, bevorzugt von 400 bis 500 °C und einem Druck von 0,05 bis 1 bar an Luft calciniert. Gelegentlich enthält das so hergestellte, im wesentlichen monokline Zircondioxid noch geringe Mengen der tetragonalen oder kubischen Modifikation. Den Anteil an der tetragonalen bzw. kubischen Modifikation kann man bis zur röntgenographischen Nachweisgrenze reduzieren, wenn vor der Calcinierung eine Trocknung unter einem Wasserdampfpartialdruck von 0,2 bis 0,9 bar durchgeführt wird. Die Trocknung erfordert beispielsweise bei 120°C ca. 16 Stunden.

Üblicherweise wird dem Zircondioxidpulver und der Organosiliciumverbindung Wasser zugesetzt, um eine knetbare Masse zu erhalten.

Ferner kann der Katalysatorträgermasse eine Säure zugesetzt werden. Diese bewirkt eine Peptisierung der Knetmasse. Geeignete Säuren sind beispielsweise Salpetersäure und Essigsäure, bevorzugt ist Salpetersäure.

Die Katalysatorträgermasse enthält üblicherweise einen Porenbildner. Geeignete Porenbildner sind beispielsweise Polyalkylenoxide wie Polyethylenoxid, Kohlenhydrate wie Cellulose und Zucker, Naturfasern, Pulp oder synthetische Polymere wie Polyvinylalkohol.

Die Katalysatorträger-Formmasse kann ferner weitere Zuschlagstoffe enthalten. Weitere Zuschlagstoffe sind beispielsweise bekannte, die Rheologie beeinflussende Verbindungen.

Die Komponenten a) bis f) werden in üblichen Mischapparaturen vermischt und homogenisiert. Geeignete Mischapparaturen sind beispielsweise Kneter, Kollergänge, Mix-Muller, die eine gute Durchmischung und Homogenisierung der anfangs inhomogenen knetbaren Masse gewährleisten. Anschließend wird die Katalysatorträger-Formmasse zu Formkörpern geformt, beispielsweise durch Extrudieren zu Strängen oder Hohlträgern.

Die Katalysatorträger-Formkörper werden üblicherweise anschließend getrocknet. Die Trocknung wird beispielsweise bei einer Temperatur von 90 bis 120 °C über einen Zeitraum von 10 bis 100 Stunden durchgeführt.

Anschließend wird der getrocknete Katalysatorträger-Formkörper calciniert. Die Calcinierung wird üblicherweise bei einer Temperatur von 300 bis 800 °C, bevorzugt von 400 bis 600 °C über einen Zeitraum von 0,5 bis 6 Stunden durchgeführt. Die Calcinierung wird vorzugsweise an Luft und bei Atmosphärendruck durchgeführt.

Die Katalysatorträger eignen sich für die Herstellung von Hydrier- und Dehydrierkatalysatoren, wie sie beispielsweise in DE-A 196 54 391 beschrieben sind. Diese enthalten auf dem Katalysatorträger ein oder mehrere Elemente, ausgewählt aus der Gruppe bestehend aus den Elementen der VIII. und/oder VI. Nebengruppe, und gegebenenfalls ein oder mehrere weitere Elemente, ausgewählt aus der Gruppe bestehend aus den Elementen der I. und II. Hauptgruppe, der III. Nebengruppe einschließlich der Lanthaniden, der III. Hauptgruppe, Rhenium, Zink und Zinn.

Als dehydrieraktive Elemente sind insbesondere Metalle der VIII. Nebengruppe geeignet, vorzugsweise die Edelmetalle Platin und Palladium, besonders bevorzugt Platin.

Wenn ein Edelmetall als dehydrieraktives Element eingesetzt wird, können zusätzliche Metalle, die das Sintern des Edelmetalls verlangsamen, wie Re und/oder Sn vorhanden sein.

Als weitere Elemente kommen solche in Frage, von denen bekannt ist, daß sie die Acidität der Katalysatoroberfläche beeinflussen oder Edelmetalle gegen Sintern stabilisieren können. Solche weiteren Elemente sind Elemente der I. und II. Hauptgruppe, nämlich Li, Na, K, Rb, Cs, Mg, Ca, Sr und Ba sowie Elemente der III. Nebengruppe wie insbesondere Y und La, einschließlich der Lanthaniden. Als wirksam hat sich auch Zn erwiesen.

Anstelle eines Edelmetalls können auch dehydrieraktive Metalle der VI. Nebengruppe, insbesondere Chrom oder Molybdän, auf dem Katalysatorträger vorliegen.

Das oder die dehydrieraktive(n) Metalle werden in der Regel durch Tränkung mit geeigneten Verbindungen der betreffenden Metalle aufgebracht. Geeignete Verbindungen sind solche, die sich durch Calcinieren in das entsprechende Metalloxid umwandeln lassen. Das oder die Metallverbindung(en) können auch aufgesprüht werden. Geeignete Metallsalze sind beispielsweise die Nitrate, Acetate und Chloride der Metalle, möglich sind auch komplexe Anionen der Metalle. Vorzugsweise werden H₂PtCl₆ oder Pt(NO₃)₂ zum Aufbringen von Platin und Cr(NO₃)₃ oder (NH₄)₂CrO₄ zum Aufbringen von Chrom eingesetzt. Zum Aufbringen von Alkali- und Erdalkalimetallen setzt man zweckmäßigerweise wässrige Lösungen von Verbindungen ein, die sich durch Calcinieren in die entsprechenden Oxide umwandeln lassen. Geeignet sind zum Beispiel Hydroxide, Carbonate, Oxalate, Acetate oder basische Carbonate der Alkali- bzw. Erdalkalimetalle. Wird der Katalysatorträger mit Metallen der III. Haupt- oder Nebengruppe dotiert, so werden häufig die Hydroxide, Carbonate, Nitrate, Acetate, Formiate oder Oxalate eingesetzt, die sich durch Calcinieren in die entsprechenden Oxide umwandeln lassen, beispielsweise La(OH)₃, La₃(CO₃)₂, La(NO₃)₃, Lanthanacetat, Lanthanformiat oder Lanthanoxalat.

Die Calcinierung der mit dem betreffenden Metallsalzlösungen imprägnierten erfindungsgemäßen Katalysatorträger erfolgt üblicherweise bei einer Temperatur von 350 bis 650°C über einen Zeitraum von 0,5 bis 6 Stunden.

Gegenstand der vorliegenden Erfindung sind auch die unter Einsatz der Katalysatorträger erhältlichen Katalysatoren. Diese werden vorzugsweise als Hydrierkatalysatoren oder Dehydrierkatalysatoren verwendet. Besonders bevorzugt ist die Verwendung der erfindungsgemäßen Katalysatoren zur Dehydrierung von Propan zu Propen.

Die Erfindung wird durch die nachstehenden Beispiele näher erläutert.

### Beispiele

### Herstellung der Katalysatorträger

### Beispiel 1

200 g ZrO₂-Pulver, das zuvor 3 Stunden lang bei 450 °C getempert wurde, werden mit 41,7 g 3-Methacryloxypropyltrimethoxysilan (Silan MEMO der Fa. Sivento), 6 g Zusoplast PS1 (Fa. Zschimmer & Schwarz), 6 ml 65 gew.-%iger HNO₃ und 92 ml Wasser 30 Minuten lang geknetet. Die erhaltene teigige Masse wird in einem Kolbenextruder zu Strängen mit 3 mm Außendurchmesser geformt. Die Stränge werden bei 120 °C getrocknet und anschließend 4 Stunden lang bei 560 °C calciniert. Der erhaltene Träger weist eine BET-Oberfläche von 109 m²/g und eine Porosität von 0,48 ml/g (gemessen mit Quecksilber-Porsimetrie) auf und besitzt eine bimodale Porendurchmesserverteilung mit Maxima bei 20 und 1100 nm. Die Schneidehärte des Trägers beträgt 25 N.

### Beispiel 2

3680 g ZrO₂-Pulver, das zuvor 3 Stunden lang bei 450 °C getempert wurde, werden mit 262,6 g Methoxysilan (Silres MSE 100 der Fa. Wacker), 110,4 g Polyethylenoxid (Alkox E100), 110,4 g 65 gew.-%iger HNO₃ und 1270 g Wasser 20 Minuten lang in einem Kollergang vermischt. Die resultierende teigige Masse wird in einem Schneckenextruder zu Strängen mit 3 mm Außendurchmesser geformt. Die Stränge werden bei 120 °C getrocknet und anschließend 4 Stunden lang bei 560 °C getempert. Der erhaltene Träger weist eine BET-Oberfläche von 95 m²/g und eine Porosität von 0,36 ml/g (gemessen mit Quecksilber-Porosimetrie) auf und besitzt eine bimodale Porendurchmesserverteilung mit Maxima bei 20 und 450 nm. Die Schneidehärte des Trägers beträgt 35 N.

### Beispiel 3

200 g ZrO₂-Pulver das zuvor 3 Stunden bei 450 °C getempert wurde, werden mit 6 g Polyethylenoxid (Alkox E100), 10,1 g Aerosil 200 (Firma Degussa), 6 ml 65 gew.-%iger HNO₃ und 100 ml Wasser 30 Minuten lang geknetet. Die erhaltene teigige Masse wird in einem Kolbenextruder zu Strängen mit 3 mm Außendurchmesser geformt. Die Stränge werden bei 120 °C getrocknet und anschließend 4 Stunden lang bei 560 °C calciniert. Der erhaltene Träger weist eine BET-Oberfäche von 75 m²/g, eine Porosität, gemessen mittels Hg-Porosimetrie, von 0,49 ml/g und eine Schneidhärte von 22 N auf

### Herstellung der Katalysatorvorläufer

### Beispiel 4

Das gemäß Beispiel 3 hergestellte Trägermaterial wird gesplittet und es wird eine Siebfraktion von 1,6 bis 2 mm erhalten. Der Trägersplitt wird mit den Aktivkomponenten Pt/Sn/K/Cs und La nach der im folgenden beschriebenen Methode beschichtet.

0,1814 g H₂PtCl₆ • 6 H₂O und 0,2758 g SnCl₂ • 2 H₂O werden in 138 ml Ethanol gelöst und in einem Rotationsverdampfer zu 23 g des ZrO₂/SiO₂-Trägermaterials aus Beispiel 3 gegeben. Das überstehende Ethanol wird am Rotationsverdampfer im Wasserstrahlpumpenvakuum (20 mbar) bei einer Wasserbadtemperatur von 40 °C abgezogen. Anschließend wird jeweils unter stehender Luft zunächst 15 Stunden lang bei 100°C getrocknet und anschließend bei 560 °C 3 Stunden lang calciniert. Danach wird der getrocknete Feststoff mit einer Lösung von 0,1773 g CsNO₃, 0,3127 g KNO₃ und 2,2626 g La(NO₃)₃ • 6 H₂O in 55 ml H₂O übergossen. Das überstehende Wasser wird am Rotationsverdampfer im Wasserstrahlpumpenvakuum (20 mbar) bei einer WasserbadTemperatur von 85 °C abgezogen. Anschließend wird jeweils unter stehender Luft zunächst 15 Stunden lang bei 100°C getrocknet und anschließend bei 560 °C 3 Stunden lang calciniert. Der so erhaltene Katalysatorvorläufer wird im folgenden als Katalysatorvorläufer 1 bezeichnet.

### Beispiel 5

Das gemäß Beispiel 2 hergestellte Trägermaterial wird gesplittet und es wird eine Siebfraktion von 1,6 bis 2 mm erhalten. Der Trägersplitt wird mit den Aktivkomponenten Pt/Sn/K/Cs und La nach der im folgenden beschriebenen Methode beschichtet:
0,2839 g H₂PtCl₆ • 6 H₂O und 0,4317 g SnCl₂ • 2 H₂O werden in 216 ml Ethanol gelöst und in einem Rotationsverdampfer zu 36 g des ZrO₂/SiO₂-Trägermaterials aus Beispiel 2 gegeben. Das überstehende Ethanol wird am Rotationsverdampfer im Wasserstrahlpumpenvakuum (20 mbar) bei einer Wasserbadtemperatur von 40 °C abgezogen. Anschließend wird jeweils unter stehender Luft zunächst 15 Stunden lang bei 100°C getrocknet und anschließend bei 560 °C 3 Stunden lang calciniert. Danach wird der getrocknete Feststoff mit einer Lösung von 0,2784 g CsNO₃, 0,4894 g KNO₃ und 3,5399 g La(NO)₃ • 6 H₂O in 86 ml H₂O übergossen. Das überstehende Wasser wird am Rotationsverdampfer im Wasserstrahlpumpenvakuum (20 mbar) bei einer WasserbadTemperatur von 85 °C abgezogen. Anschließend wird jeweils unter stehender Luft zunächst 15 Stunden lang bei 100 °C getrocknet und anschließend bei 560 °C 3 Stunden lang calciniert. Der so erhaltene Katalysatorvorläufer wird im folgenden als Katalysatorvorläufer 2 bezeichnet.

### Katalysatoraktivierung

Die Aktivierung der in den Beispielen 4 und 5 hergestellten Katalysatoren zur Dehydrierung von Propan wird in einem Laborreaktor unter den folgenden Bedingungen vorgenommen:
Ein vertikal stehender Rohrreaktor wird mit jeweils 20 ml der Katalysatorvorläufer 1 und 2 beschickt (Reaktorlänge: 520 mm; Wanddicke: 2 mm; Innendurchmesser: 20 mm; Reaktormaterial: innen-alonisiertes, d.h. mit Aluminiumoxid beschichtetes Stahlrohr; Beheizung: elektrisch (Ofen BASF Eigenbau) auf einer längsmittigen Länge von 450 mm; Länge der Katalysatorschüttung: 60 mm; Position der Katalysatorschüttung: auf der Längsmitte des Rohrreaktors; Befüllung des Reaktorrestvolumens nach oben und unten mit Steatitkugeln (Inertmaterial) eines Durchmessers von 4 bis 5 mm, nach unten auf einem Kontaktstuhl aufsitzend).

Anschließend wird das Reaktionsrohr bei einer Außenwandtemperaturregelung längs der Heizzone von 500 °C (bezogen auf ein mit einem identischen Inertgasstrom durchströmtes Rohr) mit 9,3 N1/h Wasserstoff während 30 Minuten beschickt. Anschließend wird der Wasserstoffstrom bei gleichbleibender Wandtemperatur zunächst während 30 Minuten durch einen 23,6 N1/h betragenden Strom aus 80 Vol.-% Stickstoff und 20 Vol.-% Luft und danach während 30 Minuten durch einen identischen Strom aus reiner Luft ersetzt. Unter Beibehaltung der Wandtemperatur wird dann mit einem identischen Strom von N₂ während 15 Minuten gespült und abschließend nochmals während 30 Minuten mit 9,3 N1/h Wasserstoff reduziert. Damit ist die Aktivierung des Katalysatorvorläufers abgeschlossen.

### Dehydrierung von Roh-Propan

Die katalytische Testung der aktivierten, in den Beispielen 4 und 5 hergestellten Katalysatorvorläufer 1 und 2 erfolgte jeweils im Anschluß an die Aktivierung des Kontaktes in dem gleichen Reaktor mit einem Gemisch aus 20 N1/h Roh-Propan, 18 g/h Wasserdampf und 1 N1/h Stickstoff. Dabei wurde Roh-Propan mittels eines Massendurchflußreglers der Fa. Brooks zudosiert, während das Wasser mittels einer HPLC-Pumpe (Fa. Bischoff) zunächst flüssig in den Verdampfer dosiert, in selbigem verdampft und dann mit dem Roh-Propan und Stickstoff vermischt wurde. Die Gasmischung wurde anschließend über den Kontakt geleitet. Die Wandtemperatur betrug 622 °C.

Mittels einer sich am Reaktorausgang befindlichen Druckregelung (Fa. REKO) wurde der Ausgangsdruck des Reaktors auf 1,5 bar absolut eingestellt.

Das hinter der Druckregelung auf Normaldruck entspannte Produktgas wurde abgekühlt, wobei der enthaltene Wasserdampf auskondensierte. Das nicht kondensierte Restgas wurde mittels GC (HP 6890 mit Chem-Station, Detektoren: FID, WLD, Trennsäulen: Al₂O₃/KCl (Chrompack), Carboxen 1010 (Supelco)) analysiert. In entsprechender Weise war auch das Reaktionsgas analysiert worden.

Die nachfolgenden Tabellen weisen die erzielten Ergebnisse in Abhängigkeit von der Betriebsdauer aus. Dabei beziehen sich die Angaben in Vol-% auf "trocken" gerechnetes Gas, d.h. die enthaltene Menge an Wasserdampf blieb in allen Fällen unberücksichtigt.

**Tabelle 1:**

| Aktivierter Katalysatorvorläufer 1 aus Beispiel 4 (Vergleich) | | | |
|---|---|---|---|
| | Reaktionsgas Vol.-% | Produktgas (nach 1 h) Vol.-% | Produktgas (nach 9 h) Vol.-% |
| Propan | 96,408 | 39,198 | 42,764 |
| Propen | 0,014 | 24,598 | 23,741 |
| H₂ | 0 | 30,942 | 29,281 |
| N₂ | 3,5 | 2,648 | 2,875 |
| Ethan | 0,078 | 0,544 | 0,450 |
| Ethen | 0 | 0,234 | 0,258 |
| CH₄ | 0 | 0,836 | 0,631 |

**Tabelle 2:**

| Aktivierter Katalysatovorläufer 2 aus Beispiel 5 (erfindungsgemäß) | | | |
|---|---|---|---|
| | Reaktionsgas Vol.-% | Produktgas (nach 1 h) Vol.-% | Produktgas (nach 9 h) Vol.-% |
| Propan | 96,408 | 28,924 | 31,934 |
| Propen | 0,014 | 26,574 | 27,953 |
| H₂ | 0 | 38,530 | 36,011 |
| N₂ | 3,5 | 2,386 | 2,490 |
| Ethan | 0,078 | 1,159 | 0,734 |
| Ethen | 0 | 1,166 | 0,677 |
| CH₄ | 0 | 0,261 | 0,201 |

Wie den Tabellen entnommen werden kann, weist der erfindungsgemäße Katalysator eine deutlich höhere Aktivität als der Vergleichskatalysator auf.

## Patentansprüche

1. Katalysator, enthaltend auf einem Katalysatorträger ein oder mehrere Elemente ausgewählt aus der Gruppe bestehend aus den Elementen der VIII. und/oder VI. Nebengruppe und gegebenenfalls ein oder mehrere weitere Elemente ausgewählt aus der Gruppe bestehend aus den Elementen der I. und II. Hauptgruppe, der III. Nebengruppe einschließlich der Lanthaniden, der III. Hauptgruppe, Rhenium, Zink und Zinn, wobei der Katalysatorträger hergestellt ist durch Vermischen von Zirkondioxidpulver mit einer Organosiliciumverbindung als Bindemittel, gegebenenfalls einem Porenbildner, gegebenenfalls einer Säure, Wasser sowie gegebenenfalls weiteren Zuschlagstoffen zu einer knetbaren Masse, Homogenisieren der Masse, Formung der Masse zu Formkörpern, Trocknung und Calcinierung, **dadurch gekennzeichnet, dass** das Bindemittel ausgewählt ist aus monomeren Organosiliciumverbindungen der allgemeinen Formeln (I) bis (VI)
(Hal)ₓSiR₄₋ₓ (I)
(Hal)ₓSi(OR¹)₄₋ₓ (II)
(Hal)ₓSi(NR¹R²)₄₋ₓ (III)
RₓSi(OR¹)₄₋ₓ (IV)
RₓSi(NR¹R²)₄₋ₓ (V)
(R¹O)ₓSi(NR¹R²)₄₋ₓ (VI)
worin
Hal unabhängig voneinander Halogen (F, Cl, Br oder I),
R unabhängig voneinander H oder einen gegebenenfalls substituierten Al- kyl-, Alkenyl-, Alkinyl-, Cycloalkyl-, Cycloalkenyl-, Arylalkyl- oder Arylrest,
R¹, R² unabhängig voneinander H oder einen gegebenenfalls substituierten Al- kyl-, Acyl-, Arylalkyl- oder Arylrest, und
x 0 bis 4 bedeuten.

2. Katalysator nach Anspruch 1, **dadurch gekennzeichnet, dass** zur Herstellung des Katalysatorträgers
a) 50 bis 98 Gew.-% Zircondioxidpulver,
b) 2 bis 50 Gew.-% der Organosiliciumverbindung als Bindemittel,
c) 0 bis 48 Gew.-% Porenbildner, und
d) unter 0 bis 48 Gew.-% weitere Zuschlagstoffe, wobei die Summe der Komponenten a) bis d) 100 Gew.-% ergibt, Zusatz von Wasser und einer Säure zu einer knetbaren Masse vermischt werden.

3. Katalysator nach Anspruch 1 oder 2, **dadurch gekennzeichnet, dass** das Zircondioxidpulver im wesentlichen aus monoklinem Zircondioxidpulver besteht.

4. Verwendung des Katalysators nach einem der Ansprüche 1 bis 3 als Dehydrierkatalysator.

5. Verwendung nach Anspruch 4 zur Dehydrierung von Propan zu Propen.

## Claims

1. A catalyst comprising one or more elements selected from the group consisting of the elements of transition groups VIII and/or VI and optionally one or more further elements selected from the group consisting of the elements of main groups I and II, transition group III including the lanthanides, main group III, rhenium, zinc and tin on a catalyst support, where the catalyst support is produced by mixing of zirconium dioxide powder with an organosilicon compound as binder, optionally a pore former, optionally an acid, water and optionally further additives to give a kneadable composition, homogenization of the composition, shaping of the composition to give shaped bodies, drying and calcination, wherein the binder is selected from among monomeric organosilicon compounds of the general formulae (I) to (VI)
(Hal)ₓSiR₄₋ₓ (I)
(Hal)ₓSi(OR¹)₄₋ₓ (II)
(Hal)ₓSi(NR¹R²)₄₋ₓ (III)
RₓSi(OR¹)₄₋ₓ (IV)
RₓSi(NR¹R²)₄₋ₓ (V)
(R¹O)ₓSi (NR¹R²)₄₋ₓ (VI)
where
the radicals Hal are each, independently of one another, halogen (F, Cl, Br or I),
the radicals R are each, independently of one another, H or an optionally substituted alkyl, alkenyl, alkynyl, cycloalkyl, cycloalkenyl, arylalkyl or aryl radical,
R¹, R² are each, independently of one another, H or an optionally substituted alkyl, acyl, arylalkyl or aryl radical and
x is from 0 to 4.

2. The catalyst according to claim 1, wherein the catalyst support is produced by mixing
a) from 50 to 98% by weight of zirconium dioxide powder,
b) from 2 to 50% by weight of the organosilicon compound as binder,
c) from 0 to 48% by weight of pore formers and
d) from 0 to 48% by weight of further additives, where the sum of the components a) to d) is 100% by weight,
with addition of water and an acid to give a kneadable composition.

3. The catalyst according to claim 1 or 2, wherein the zirconium dioxide powder consists essentially of monoclinic zirconium dioxide powder.

4. The use of the catalyst according to any of claims 1 to 3 as dehydrogenation catalyst.

5. The use according to claim 4 for the dehydrogenation of propane to propene.

## Revendications

1. Catalyseur, contenant sur un support de catalyseur un ou plusieurs éléments choisis dans le groupe constitué par les éléments du groupe de transition VIII et/ou VI et éventuellement un ou plusieurs éléments supplémentaires choisis dans le groupe constitué par les éléments du groupe principal I et II, du groupe de transition III y compris les lanthanides, du groupe principal III, le rhénium, le zinc et l'étain, le support de catalyseur étant fabriqué par mélange d'une poudre de dioxyde de zirconium avec un composé d'organosilicium en tant que liant, éventuellement un agent de formation de pores, éventuellement un acide, de l'eau et éventuellement des additifs supplémentaires pour former une masse malaxable, homogénéisation de la masse, façonnage de la masse en corps moulés, séchage et calcination, **caractérisé en ce que** le liant est choisi parmi les composés d'organosilicium monomères de formules générales (I) à (VI)
(Hal)ₓSiR₄₋ₓ (I)
(Hal)ₓSi(OR¹)₄₋ₓ (II)
(Hal)ₓSi (NR¹R²)₄₋ₓ (III)
RₓSi(OR¹)₄₋ₓ (IV)
RₓSi (NR¹R²)₄₋ₓ (V)
(R¹O)ₓSi (NR¹R²)₄₋ₓ (VI)
dans lesquelles
Hal signifient indépendamment les uns des autres un halogène (F, Cl, Br ou I),
R signifient indépendamment les uns des autres H ou un radical alkyle, alcényle, alcynyle, cycloalkyle, cycloalcényle, arylalkyle ou aryle éventuellement substitué,
R¹, R² signifient indépendamment les uns des autres H ou un radical alkyle, acyle, arylalkyle ou aryle éventuellement substitué, et
x signifie 0 à 4.

2. Catalyseur selon la revendication 1, **caractérisé en ce que** pour la fabrication du support de catalyseur
a) 50 à 98% en poids de poudre de dioxyde de zirconium,
b) 2 à 50% en poids du composé d'organosilicium en tant que liant,
c) 0 à 48 % en poids d'agent de formation de pores et
d) 0 à 48 % en poids d'additifs supplémentaires,
la somme des composants a) à d) étant de 100% en poids,
sont mélangés pour former une masse malaxable avec ajout d'eau et d'un acide.

3. Catalyseur selon la revendication 1 ou 2, **caractérisé en ce que** la poudre de dioxyde de zirconium est essentiellement constituée d'une poudre de dioxyde de zirconium monoclinique.

4. Utilisation du catalyseur selon l'une quelconque des revendications 1 à 3 en tant que catalyseur de déshydrogénation.

5. Utilisation selon la revendication 4 pour la déshydrogénation de propane en propène.
